# EUROPEAN PATENT APPLICATION

(11) **EP 1 473 038 A1**
(43) Date of publication of application: **03.11.2004**
(21) Application number: 02790788.0
(22) Date of filing: 16.12.2002
(51) Int. Cl.: A61K 35/84, A61P 37/04, A61P 43/00

(54) **METHOD OF PROTECTING LIVING BODY FROM FOREIGN FACTOR AND COMPOSITION THEREFOR**

(30) Priority: 08.02.2002 JP 2002033256
(71) Applicant: Sundory Co., Ltd., Sakai-shi, Osaka 591-8023 (JP); Kyowa Engineering Co., Ltd., Chiyoda-ku, Tokyo 102-0074 (JP)
(72) Inventor: TAKEUCHI, Minoru, Kyoto-shi, Kyoto 607-8024 (JP); NAKAJIMA, Atsuko, Kyoto-shi, Kyoto 606-8044 (JP)
(74) Representative: Harding, Charles Thomas
(86) International application number: PCT/JP2002/013145
(87) International publication number: WO 2003/066076

(57) **Abstract**

The objective is to provide a composition or a food material for protecting a living body from an external factor, which includes an extract from *Agaricus Blazei Murill*. The composition protects a living body through immunocompetent cells. The immunocompetent cells may be macrophages, and optionally, T cells. The immunocompetent cells may be antibody producing cells. The composition may protect a living body through an enhancement of humoral immunity. The enhancement of the humoral immunity may be caused by activating lymphocytes selected from the group consisting of activating T cells and macrophages, and thus enhancing expression of interleukin selected from the group consisting of interleukin-1β and interleukin 6. The external factor may be taken by smoking, or may be an antigen substance.

## Description

### TECHNICAL FIELD

The present invention relates to a method for preventing damage of a living body which is caused by external factors and a composition for the same, including a substance obtained by processing agaricus by an extracting operation.

### BACKGROUND ART

A human is always surrounded by environment polluting substances as external factors. The environment polluting substances include organisms such as innumerable types of viruses, bacteria, molds, parasites and the like, and numerous numbers of antigen substances besides these organisms, carcinogens and carcinogenesis acceleration substances in tobacco smoke. For maintaining health, negative influences of these external factors should be eliminated.

Under such circumstances, as the desire for maintaining health increases, in general, more people tend to take health food and health supplements. Therefore, natural materials which hardly have side effects and which include an active ingredient (particularly, which have an antitumor effect) are drawing attention for use in health food or health supplements. This is because chemical medicaments and synthesized components show a number of side effects along with antitumor activity. Examples of natural materials having an active component includes, mushroom, agaricus mushroom, or the like.

A number of polysaccharides having antitumor activity are isolated from mushrooms (Hamuro J et al. (1978), Cancer Res.38:3080-3085; Mizuno T et al.(1992), Bio-sci.Biotechnol.Biochem.56:347-348).

The mushroom which is generally called agaricus belongs to the family Agaricaceae of the division Basidiomycota and is referred to by the botanical name "*Agaricus blazei* Murill" and the Japanese name "kawariharatake". *Agaricus blazei* Murill (hereinafter, generally referred to as ABM, or agaricus) has been traditionally used as a medicament in the Piedade region in Sao Paulo, Brazil. It is said that agaricus has a variety of immune activation activities, cancer prevention effects, tumor growth suppression effects, and the like. Currently, it is provided for internal use as health food.

Polysaccharides from agaricus include β-1, 6-glucopyranosyl residues and have antitumor activity against Sarcoma 180 (Ebina T et al.(1986), Jpn.J.Cancer Res 77:1034-1042). Extracts from agaricus include (1→4)-α-D-glucan having (1→6)-β branched chain, and having natural killer cell activation activity and selective antitumor activity mediated through apoptosis (Fujimiya Y et al. (1998), Cancer Immunol Immunother 46:147-159). Peptideglycans from agaricus have a direct cytotoxic activity against Meth A tumor cells in a double implanted tumor system and an indirect immune enhancement activity in tumor-bearing mice (Ebina T et al.(1998), Biotherapy 11:259-265). Polysaccharides from agaricus change the percentage of spleen Thy1,2-, L3T4 positive cells in a T cell subset of mice (Mizuno M et al. (1998), Biosci.Biotechnol.Biochem.62:434-437). These reports suggest that polysaccharides from agaricus have cytotoxic activity against tumor cells through an immunomodulation activity.

There have been many reports that say various natural materials, particularly agaricus extracts, have an immune enhancement activity and antitumor activity. However, there is no report that they enhance immune activities through delayed hypersensitiviy or antibody production. The mechanism of how agaricus extracts activate an immune system has not been solved. Further, there are hardly any reports which specifically confirm a bioactivity for agaricus extracts beside immune activation activity, carcinogenesis prevention effects and tumor growth suppression effects.

### DISCLOSURE OF THE INVENTION

A living body is always subjected to environment polluting substances as external factors. The environment polluting substances include organisms such as innumerable types of viruses, bacteria, molds, parasites and the like, and numerous numbers of antigen substances besides these organisms, carcinogens and carcinogenesis acceleration substances in tobacco smoke. It is considered that such external factors to a living body or abnormal variant cells (for example, cancer cells) generated in a living body are mainly removed by an infection protecting function and an immunologically monitoring mechanism of an immune system of the living body. Therefore, it is important to enhance an immune system of a living body for maintaining and enhancing the health of the individual. It is also an important issue, which is necessary for maintaining and enhancing health of the individual, to actually examine bioactive effects of agaricus, which is a natural material, such as various immune activation activity, carcinogenesis prevention effects, tumor growth suppression effects, immune activation effects and so on.

We completed the present invention by examining whether or not the kawariharatake extract induces antibody production, and studied the mechanism therefor. Further, we completed the present invention by allowing mice to inhale primary tobacco smoke, confirming DNA damage in lungs, alveoli pulmonis Mφ and cardiac tissue caused by smoking, and further confirming the effects of the kawariharatake extract in preventing this damage.

The present invention relates to a composition for protecting a living body from an external factor, and the composition includes an extract from agaricus.

The composition may protect a living body through activating immunocompetent cells.

The immunocompetent cells may be macrophages.

The immunocompetent cells may be macrophages or T cells.

The immunocompetent cells may be antibody producing cells.

The composition may protect a living body through an enhancement of humoral immunity.

The external factor may be taken by smoking.

The composition may protect a living body by decreasing genetic damage.

The genetic damage may be oxidative DNA damage of a pulmonary tissue.

The external factor may be an antigen substance.

The enhancement of the humoral immunity may be caused by activated lymphocytes selected from the group consisting of activating T cells and macrophages.

The enhancement of the humoral immunity may be caused by enhancing expression of interleukin selected from the group consisting of interleukin-1β and interleukin 6.

The composition may further comprise a pharmaceutically acceptable carrier.

The composition may be in a form selected from the group consisting of powder, liquid, tablet, capsule, and pellet.

The present invention further relates to a method for protecting a living body from an external factor, and the method comprises a step of administering a composition including an extract from agaricus to a subject.

The method may protect a living body through immunocompetent cells.

The immunocompetent cells may be macrophages.

The immunocompetent cells may be macrophages or T cells.

The immunocompetent cells may be antibody producing cells.

The method may protect a living body through an enhancement of humoral immunity.

The external factor may be taken by smoking.

The method may protect a living body by decreasing genetic damage.

The genetic damage may be oxidative DNA damages of a pulmonary tissue.

The external factor may be an antigen substance.

The enhancement of the humoral immunity may be caused by activated lymphocytes selected from the group consisting of activating T cells and macrophages.

The enhancement of the humoral immunity may be caused by enhancing expression of an interleukin selected from the group consisting of interleukin-1β and interleukin 6.

The present invention further relates to use of an extract from agaricus for preparation of a composition for protecting a living body from an external factor.

The term "external factor" to a living body as used herein includes: environment polluting substances including organisms such as viruses, bacteria, molds, parasites and the like, antigen substances besides these organisms, and carcinogens and carcinogenesis acceleration substances in tobacco smoke; and even abnormal variant cells (for example, cancer cells) generated in a living body. The term "protecting a living body from external factors" as used herein means that occurrence of syndromes such as viral diseases, allergies, oxidative damage of living tissue, tumor, cancer or the like caused by the "external factors" is prevented or delayed, or the syndrome is alleviated or eliminated. The term "immunocompetent cells" means cells involved in an immune response, which is known to those skilled in the art. The immunocompetent cells can be roughly divided into B cells which mediate humoral immunity and T cells which mediate cellular immunity. The immunocompetent cells include lymphocytes; accessory cells such as macrophages, Langerhans cells, dendritic cells and the like; NK cells (natural killer cells); LAK cells (lymphokine activated killer cell); and cells inducing antibody dependent cell injury; and the like. T cells include helper T cells and suppressor T cells which control immune response, killer T cells which destroy target cells, and T cells involved in delayed hypersensitivity. B cells include antibody secretion cells differentiated from B cells upon activation by an antigen or a T cell.

The agaricus extract may include, as an effective ingredient, a main fraction eluted chromatographically of a molecular weight of 100 to 2000 obtained by the steps of extraction with hot water from a fruiting body of agaricus, dialyzing the extract, and performing a chromatography process on the dialyzate.

Alternately, the agaricus extract may include, as an effective ingredient, a dialyzate obtained by the steps of extraction with hot water from a fruiting body of agaricus, mixing the obtained extract with ethanol and centrifuging to separate precipitate and supernatant, mixing the supernatant with ethanol and centrifuging to separate precipitate and supernatant, and dissolving the precipitate into diluted water to perform dialysis.

The agaricus extract may as appropriate be in the form of mixed with a pharmaceutically acceptable carrier. Pharmaceutically acceptable carriers are known to those skilled in the art and include, for example, the following carriers but not limited to these: buffers such as Ringer's solution, Hank's balanced salt solution, or buffered physiological saline; fatty acids such as sesame oil; synthetic fatty acid esters such as ethyl oleate or triglycerides; saccharides such as lactose, sucrose, mannitol, sorbitol; starches derived from vegetables such as corn, wheat, rice, or potato; cellulose such as methylcellulose, hydroxypropylmethylcellulose, or sodium carboxymethylcellulose; rubber such as gum arabic or tragacanth; proteins such as gelatin or collagen; cross-linked polyvinyl pyrrolidone, agar, alginic acid or salts thereof, or the like.

### BRIEF DESCRIPTION OF THE DRAWINGS

Figure **1** is a photograph showing the result of immunological staining in a pulmonary tissue of mice.
Figure **2** is a diagram showing the influence on 8-OHdG expression in a pulmonary tissue by smoking.
Figure **3** is a diagram showing the influence on 8-OHdG concentration in pulmonary tissue DNA of smoking and an effect of administration of the composition of the present invention.
Figure **4** is a diagram showing the influence on activated oxygen production in alveoli pulmonis Mφ cell of smoking.
Figure **5** is a diagram showing the influence on 8-OHdG concentration in alveoli pulmonis Mφ DNA of smoking and an effect of administration of the composition of the present invention.
Figure **6** is a diagram showing the influence on 8-OHdG concentration in cardiac tissue DNA of smoking.
Figure **7** is a diagram showing the influence on 8-OHdG concentration in urine of smoking and an effect of administration of the composition of the present invention.
Figure **8** is a photograph of a Hamburg II automatic smoking apparatus.

Figure **9** is a diagram showing influences of ABM extract on the number of PFCs. On the 0th day, 1×10⁸ SRBC, and ABM extract (25 mg/kg) or PBS as a control were intra-abdominally injected to mice. On the fourth day, the spleens of the mice were collected and PFCs against SRBC antigen were counted by using a PFC assay. (a) shows the number of PFCs per 10⁶ spleen cells and (b) shows the number of PFCs per spleen. The results are shown with average ± S.D. (n=12). ** indicates a statistically significant result compared to the control (p<0.01).

Figure 10 is a photograph of an electrophoresis gel showing the influence of an ABM extract on expression of IL-1β and IL-6 mRNA in spleen cells. Mice were given intra-abdominal injection with an ABM extract (25 mg/kg) (+) or without the ABM extract (-). Three or four days later, spleens were collected and total RNA was separated by using the AGPC method. Then, RT-PCR was performed 26 cycles for β-actin, 29 cycles for IL-1β, and 32 cycles for IL-6. The PCR product was subjected to 30% polyacrylamide gel electrophoresis, and then visualized using ethidium bromide.

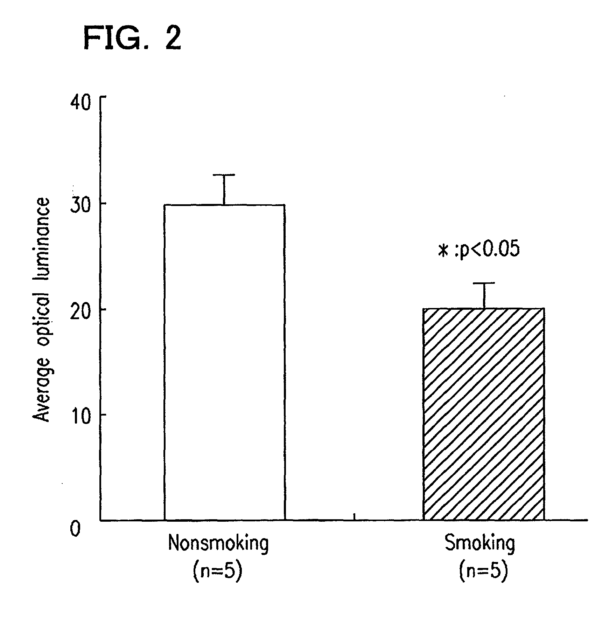

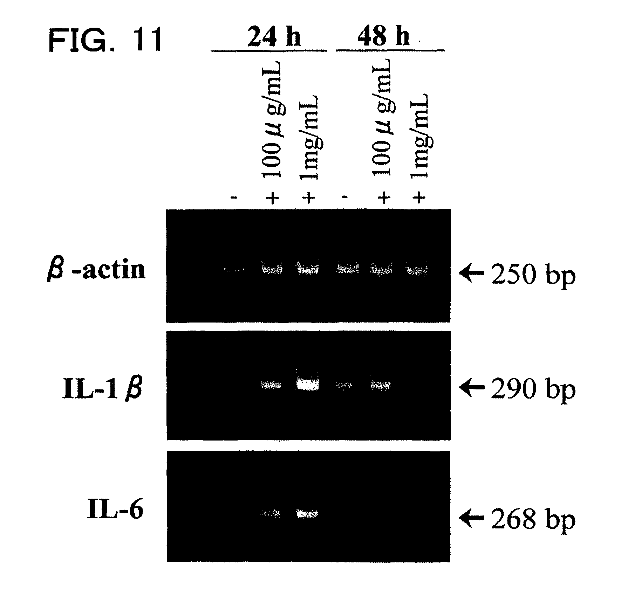

Figure 11 is a photograph of an electrophoresis gel showing an influence of an ABM extract on expression of IL-1β and IL-6mRNA in abdominal macrophages. Abdominal cells were distributed in a flat-bottomed culture plate of 96 wells (5×10⁴ cells/0.1 ml/well), with or without 0.1 or 1 mg/mL ABM extract. The cells were incubated at 37°C in 5% CO₂ for 24 or 48 hours. Then, the total RNA was separated using an AGPC method. Next, RT-PCR was performed as described with respect to Figure **2**. The PCR product was subjected to 30% polyacrylamide gel electrophoresis, and then visualized using ethidium bromide.

### BEST MODE FOR CARRYING OUT THE INVENTION

Hereinafter, a method for producing a composition which prevents damage of a living body caused by external factors according to the present invention is described.

The agaricus used in the present invention may be a fruiting body or mycelium portion of natural or artificially-cultured agaricus. For convenience, commercially available dried fruiting bodies can also be used. The agaricus is used as it is, is cut, or is powdered and provided for preparing an agaricus extract.

The agaricus extract is an extract of agaricus including an agaricus-derived ingredient obtained by using water, lower alcohols, or the like as a solvent. Typically, water, ethanol, aqueous ethanol, or the like is used. Any solvent can be used as long as a damage of a living body which is caused by an external factor can be prevented. In general, the dried fruiting body, or the powder thereof, is mixed with a solvent of 2 through 10 times the weight thereof to perform extraction. Examples of solvents include water, ethanol, propanol, butanol, acetone, 1,3-butylene glycol, ethyl acetate, hexane, methylene chloride, methanol, or a mixture thereof.

Typically, the agaricus extract can be obtained by using water as a solvent. It is prepared by extraction with hot water from agaricus. Extraction from agaricus with hot water may be performed by mixing dried fruiting body/bodies with 5 through 10 times the weight thereof of water, and applying heat to the extract or heat-refluxing the mixture for 1 through 3 hours. As necessary, hot water extraction from agaricus may be performed by repeating the hot water extraction or the heat-reflux on a residue previously extracted with hot water. The solution extracted with hot water thus obtained is dried by a method known to those skilled in the art such as lyophilization, spray-drying, or the like to obtain a dried product (hereinafter, referred to as dried product A). Dried product A is mixed with 5 through 20 times the weight thereof of water. Then, the solution is put into a dialysis tube and dialyzed for 10 through 15 hours with several times the amount thereof of distilled water. The obtained dialyzate is lyophilized to obtain a dried product of the agaricus extracted with hot water (hereinafter, referred to as dried product C).

Then, the solution remaining in the dialysis tube is further dialyzed against running water for 20 through 40 hours and dialyzed twice against distilled water for a few hours each time and a dried product of the solution remaining in the dialysis tube is obtained as described above. Thus, the dried product of the agaricus extracted with hot water (hereinafter, referred to as dried product B), which prevents damage of a living body caused by external factors, can be obtained.

Next, obtained dried product C is dissolved into about ten times the weight thereof of distilled water. Chromatography is performed with distilled water as an eluent to obtain 20 mL fractions. From the obtained fractions, a main ingredient in the middle fractions which has a molecular weight of about 100-2000 Da by gel filtration is a fraction of the agaricus extracted with hot water, which prevents damage of a living body caused by external factors.

These fractions are analyzed further using reverse-phase chromatography which uses ODS (octadecyl silanated silica gel), ion-exchange chromatography using DEAE-TOYOPEARL 650, or the like, and confirmed to include a plurality of ingredients such as arginine, lysine, mannitol, and the like.

The solution extracted with hot water, obtained by the above-described method, is mixed with an equal amount of ethanol. The mixture is centrifuged to separate precipitate and supernatant. The obtained supernatant is further mixed with ethanol of 1 through 3 volumes thereof. The mixture is further centrifuged to obtain precipitate. The precipitate obtained is dissolved in distilled water and the solution obtained is dialyzed. The dialyzate obtained is also a low-molecular weight fraction of the agaricus extracted with hot water, which prevents damage of a living body caused by external factors, according to the present invention.

The agaricus extract obtained as described above or fractions thereof can be used for production of medicines by themselves or in combination with various carriers. Further, the extract of agaricus or the fractions thereof may be used as health foods by themselves or by using with other foods.

Typically, the composition of the claimed invention can be taken orally with a biocompatible pharmaceutical carrier (for example, physiological saline, buffered physiological saline, dextrose, water, or the like). The compositions of the present invention can be taken by itself or in combination with other medicines or food materials.

The composition of the present invention may include a pharmaceutically acceptable carrier. Examples of the pharmaceutically acceptable carrier include, for example, physiological saline, buffered physiological saline, dextrose, water, or the like. In general, the pharmaceutically acceptable carrier is pharmaceutically inactive.

The composition of the present invention may also include agaricus extracts or a fraction thereof, an excipient, an adjuvant, and a pharmaceutically acceptable carrier. Further, the composition may be combined to other medicines in addition to the agaricus extracts or a fraction thereof and administered to a patient.

The compositions of the present invention can be administered orally or parenterally. Parenteral administration includes topical, dermal, intra-arterial, intramuscular, subcutaneous, intramedullary, intra-cisternal, intraventricular, intravenous, intra-abdominal, or intranasal administration. Preferably, the compositions of the present invention are administered by intravenous or intra-arterial injection. The technique for formulation and administration of the pharmaceutical composition is well-known to those skilled in the art as described in a textbook well-known to those skilled in the art, "REMINGTON'S PHARMACEUTICAL SCIENCES" (Maack Publishing Co., Easton, PA).

The composition of the present invention includes the agaricus extract or the fraction thereof in a pharmaceutically effective amount for preventing damage of a living body caused by external factors. Typically, the composition of the present invention may be prepared for administration such that a dosage of the agaricus extract or the fraction thereof will be about 5 mg/kg of body weight to about 100 mg/kg of body weight. The term "pharmaceutically effective amount" can be sufficiently recognized by those skilled in the art, and refers to an amount of agaricus extract or the fraction thereof which is effective for preventing an intended damage of a living body which is caused by an external factor in a living body. Thus, the pharmaceutically effective amount is an amount sufficient for alleviating symptoms of a damage of a living body caused by external factors, which should otherwise be treated.

An example of assays useful for confirming the "pharmaceutically effective amount" is to measure a degree of damage of a living body, which is caused by an external factor, for example, an oxidative DNA damage in a subject. The amount of the agaricus extract or the fraction thereof which is actually administered depends on the health conditions, or the like of the individual to which the extract is applied and may be optimized so that a desirable effect can be achieved. It is a routine process for those skilled in the art to determine a pharmaceutically effective amount.

The pharmaceutically effective amount can be first evaluated by *in vitro* assay using cell culture or suitable animal models. Then, based on the obtained information, an amount and a route which are effective in administration to a human can be determined.

A composition for oral administration can be formulated as a composition including a pharmaceutically acceptable carrier well known in the art in a prescription form suitable for administration. Such a carrier allows the composition obtained to be formulated as a tablet, pill, sugar-coated pill, capsule, liquid, gel, syrup, slurry, suspension, or the like, suitable for ingestion by patients.

The agaricus extract and the fraction thereof having the activity of preventing damage of a living body caused by external factors can be mixed with one or more selected food materials in an amount sufficient for exerting its function. The one or more selected food materials are mixed with the fraction having immune activation activity in a form known to those skilled in the art, usually, powder form. The mixture can be served as a liquid food product depending on its utility or on preference. Alternatively, the mixture maybe formed as capsules such as hard capsules or soft capsules, tablets, or pills, or may be formed into a powdery, granular, tea-leaf, tea-bag, or candy form.

Hereinafter, the present invention will be further described by way of examples. The following examples are merely illustrative and do not limit the present invention.

### Examples

### (Example 1) Effects of agaricus hot water extract on DNA damage caused by smoking

### I. Materials and methods

Materials and methods used in Example 1 are as follows.

### 1. Smoking

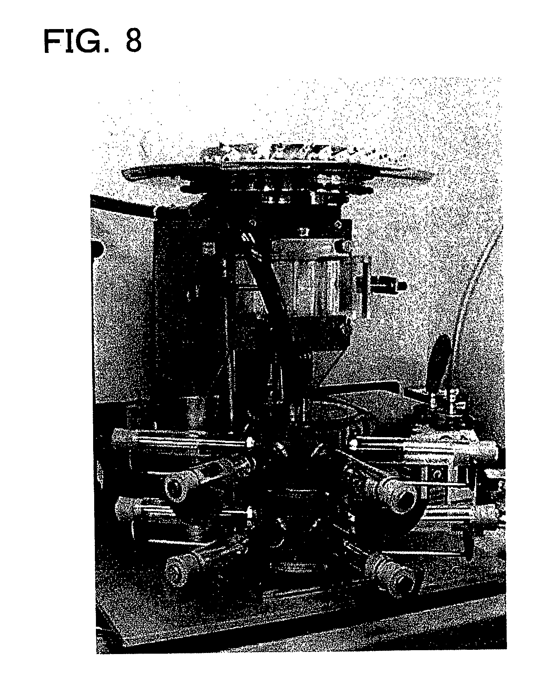

Mice (8 week-old C57BL/6 mice) were made to smoke 20 cigarettes (CORESTA: which included 15 mg of tar and 1.5 mg of nicotine per 1 cigarette) a day for 10 days, by using a Hamburg II automatic smoking apparatus (available from Borgwaldt Corp.) shown in Figure 8.

### 2. Agaricus extract

The hot water extract from agaricus (ABM) (dried product A mentioned above) was supplied from Kyowa Engineering Co. (Tokyo, Japan). This was extracted from ABM by the method described in Mizuno et al (ibid) with some modification. Specifically, extraction of a dried fruiting body of ABM was performed using boiling water. The resultant solution was centrifuged for 10 minutes at 1800xg and lyophilized to obtain the agaricus hot water extract. Then, the product was again dissolved in Dulbecco's buffered physiological saline (PBS) free of Ca⁺⁺ and Mg⁺⁺ (Nissui Pharmaceutical Corp., Tokyo, Japan) at a concentration of 10 mg/mL. The solution was filtered with a 0.45 µm membrane filter (Millipore Co. Ltd, Bedford, MA). The obtained filtrate was stored at 4°C until use. The filtrate was intravenously injected (i.p.) before smoking such that administration would be 25 mg/ kg of body weight. A control group was given only PBS without the agaricus hot water extract.

### 3. Alveoli pulmonis macrophage (Mφ)

On the first day after the smoking was finished, the BAL (Broncho alveolar lavage) method was performed to collect BAL liquid. In the BAL method, an operation of inserting 1 ml of PBS with a syringe into a main bronchus of mice killed with an anesthetic using ether and aspirating the liquid was repeated 10 times. Then, the obtained BAL liquid was centrifuged at 1000 rpm for 10 minutes. The obtained precipitate was collected as Mφ.

### 4. Measurement of 8-OHdG (8-Hydroxy deoxy guanosine) in pulmonary tissue

On the first day after the smoking was finished, a pulmonary tissue was removed, and then a paraffin section was prepared in accordance with a routine method. The section was immunologically stained with OAB (dimethylaminoazobenzene) dyes. The intensity of staining was observed under a microscope to measure 8-OHdG (8-Hydroxy deoxy guanosine).

### 5. 8-OHdG in DNA in lung, alveoli pulmonis Mφ, and heart

Tobacco smoke includes a number of carcinogens and carcinogenesis accelerating substances, such as benzpyrene. It has been reported that smoking is related to incidence of lung cancer and 8-OHdG is involved in carcinogenesis (Floyd, R.A.Carcinogenesis, 11:1447-1450, 1990).

On the first day after the smoking was finished and the tissues were extracted, DNA was extracted in accordance with a routine method (the method of Lu Wang et al.). Then, the measurement was performed using the ELISA method which uses an anti-8-OHdG antibody as a primary antibody and IgG enzyme-labeled antibody as a secondary antibody. The alveoli pulmonis Mφ was collected by performing bronchus alveoli pulmonis washing of the excised lung. Further, urine was collected on the first day after the smoking was finished. 8 -OHdG was also measured also in the urine by the ELISA method.

### 6. Production of O₂⁻ and H₂O₂ in lung, alveoli pulmonis Mφ, and heart

The alveoli pulmonis Mφ were collected on the first day after smoking was finished as described in section 3 above. Then, 2.5 µg/ml of Hydroethidine and 20 µg/ml of DCFH-DA (dichlorofluorescin diacetate) were added thereto. The mixture was shaken for 30 minutes at 37°C for reaction. Mφ in the reaction solution were washed by a centrifuging operation. The resultant mixture was measure using FACSort (Bass, D.A. et al., J.Immunol.139:1910-1917, 1983).

### II. Results

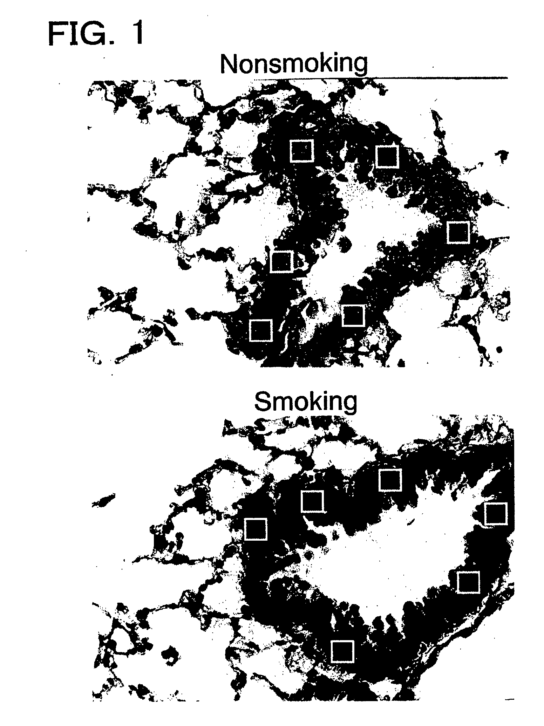

Figure **1** shows the result of immunological staining in the pulmonary tissue. The upper half of Figure **1** is a micrograph showing the result of immunological staining in the pulmonary tissue of the nonsmoking mice. The lower half of Figure **1** is a micrograph showing the results of immunological staining in the pulmonary tissue of the smoking mice. As shown in Figure **1**, stronger staining is recognized in the smoking mice compared to the nonsmoking mice.

Next, 6 sites of the epidermis of the pulmonary bronchus of the lungs of the respective mice (the portions indicted by squares in Figure **1**) were selected at random. Average optical luminance of immunological staining was analyzed using Image-Pro Plus (Planetron, Inc.). Figure **2** shows the results. As shown in Figure **2**, the average luminance of the nonsmoking group was significantly higher than the average luminance of the smoking group.

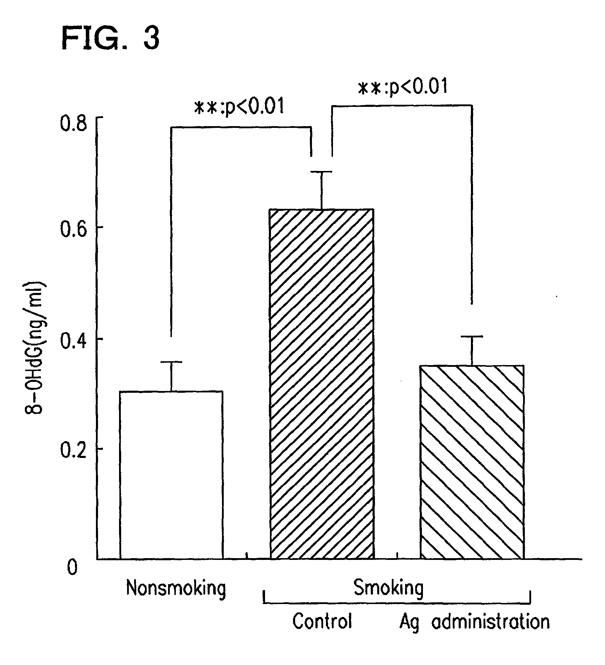

Figure **3** shows the measurement results of 8-OHdG in DNA of pulmonary tissue. As shown in Figure **3**, 8-OHdG value in the pulmonary tissue DNA is recognized to be significantly higher in the smoking group than in the nonsmoking group. However, the value significantly lowers by the administration of the agaricus extract (referred to as Ag in Figure 3 and the following figures).

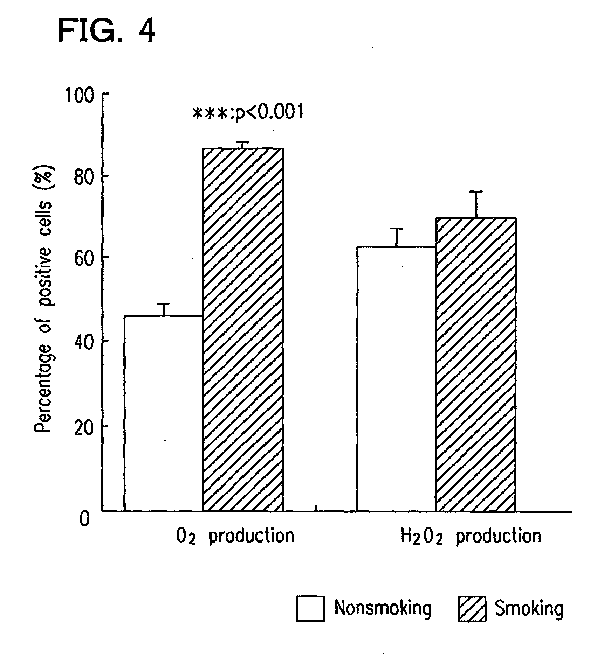

Figure **4** shows the measurement results of O₂⁻ and H₂O₂ in alveoli pulmonis Mφ. As illustrated, the percentage of O₂ production positive cells in alveoli pulmonis Mφ is significantly higher in the smoking group than in the nonsmoking group. It is recognized that H₂O₂ production cells tend to be higher in the smoking group than in the nonsmoking group.

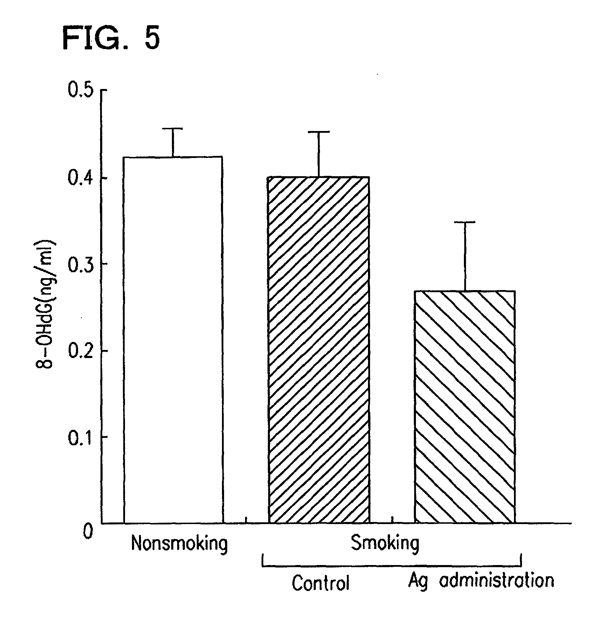

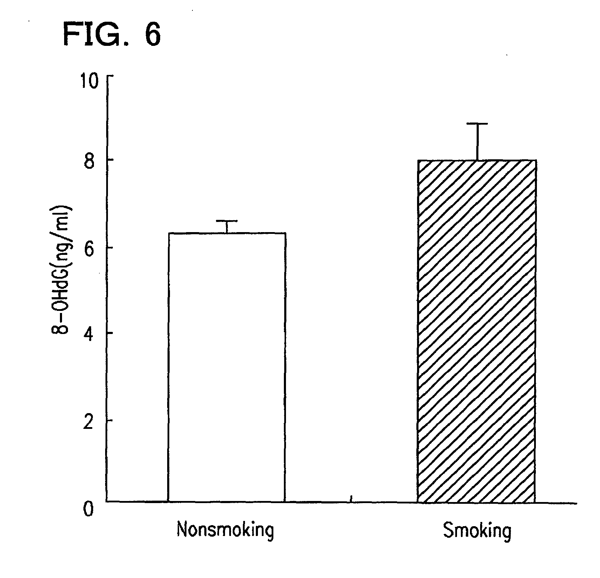

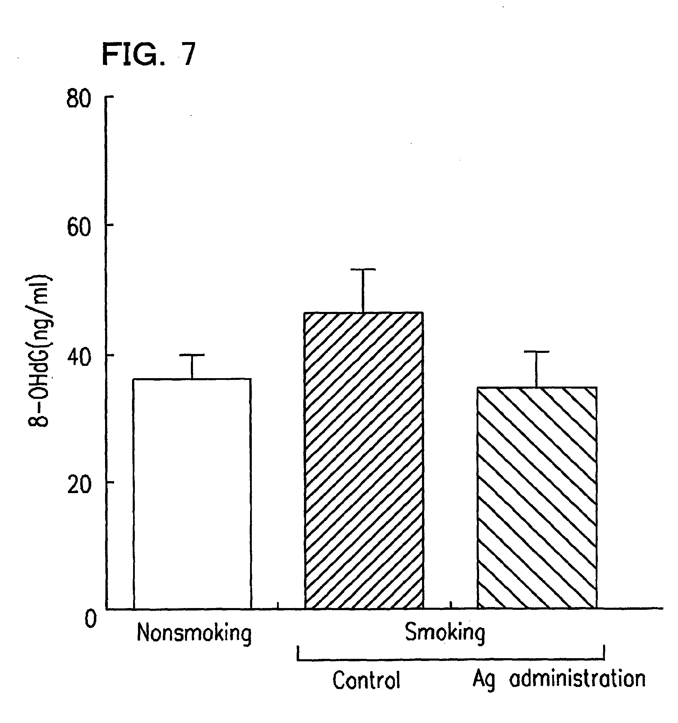

Figures **5, 6** and **7** showmeasurement results of 8-OHdG respectively in alveoli pulmonis Mφ DNA, cardiac tissue DNA, andurine. As shown in the figures, no significant difference between the smoking group and the nonsmoking group is observed for 8-OHdG values in alveoli pulmonis Mφ DNA, cardiac tissue DNA, and urine. However, it is recognized that 8-OHdG values in alveoli pulmonis Mφ DNA and urine tend to lower by administrating the agaricus hot water extract (referred to as Ag in Figures **5** and **7**).

Based on the above-mentioned results, it is shown that, as the production of O₂⁻ in alveoli pulmonis Mφ increases due to smoking, an oxidative DNA damage is caused in the pulmonary tissue and the administration of the agaricus extract has an effect of preventing an oxidative damage which is caused by smoking.

### (Example 2) Effect of agaricus hot water extract on antibody production

### I. Materials and methods

Materials and methods used in Example 2 are as follows.

### 1. Mice

Female C57BL/6 mice (Japan SLC Inc., Hamamatsu, Japan) of 8-10 week-old were used. 12 mice were divided into an agaricus (*Agaricus blazei Murill*, hereinafter, abbreviated as ABM) group and a control group. They were allowed to freely take a standard non-purified food (8.7 g water, 25.2 g crude protein, 4.6 g crude lipid, 4.4 g crude fiber, 6.5 g crude minerals, 50.7 g NFE/100 g of food: 344.4 kcal; Clea Japan, Osaka, Japan) and water. The mice were kept in a room which was maintained at 25°C. Mice were handled in accordance with the ethical guidelines of the animal committee of Kyoto Sangyo University.

### 2. Preparation of agaricus extract

The hot water extract from agaricus (ABM) was supplied from Kyowa Engineering Co. (Tokyo Japan). This was extracted from ABM by the method described in Mizuno et al (ibid) with some modification. Specifically, extraction of a dried fruiting body of ABM was performed using boiling water. The resultant solution was centrifuged for 10 minutes at 1800xg and lyophilized to obtain the agaricus hot water extract. Then, the product was again dissolved in Dulbecco's buffered physiological saline (PBS) free of Ca⁺⁺ and Mg⁺⁺ (Nissui Pharmaceutical Corp., Tokyo, Japan) at a concentration of 10 mg/mL. The solution was filtered with a 0.45 µm membrane filter (Millipore Co. Ltd, Bedford, MA). The obtained filtrate was stored at 4°C until use.

### 3. Assay of plaque forming cell (PFC)

Sheep red blood cells (SRBC) were used as antigen and were obtained from Nikken Bio-medical laboratory Inc. (Kyoto, Japan). SRBC were washed with physiological saline three times. 1×10⁸ SRBC with ABM abstract (25 mg/kg) or PBS as a control were intra-abdominally injected to mice. Four days later, mice were killed. In accordance with the method described in Cunnigham, A.J. et al., Immunology 14:599-600, 1968, spleens thereof were excised, cut on a steel mesh, and passed through a layer of gauze to obtain a suspension of spleen cells. Then, the obtained suspension was centrifuged twice at 490xg for five minutes. The obtained pellets were again suspended into MEM medium of 10 mL of Eagle's medium (Nikken Bio-medical laboratory Inc.(Kyoto, Japan)). A count of the surviving cells was performed by a Trypan Blue clearance test. The number of PFC with respect to SRBC antigen was determined by the method of Cunningham and Szenberg ( Cunningham AJ et al . (1968), Immunology. 14(4): 599-600). Specifically, 100 µL of the aliquot of the spleen cell suspension was mixed with 50 µL of 50% SRBC and 24% complement so as to obtain the final volume of 500 µL. 100 µL of the sample was incubated at 37°C for one hour in Cunnigham's chamber (trademark) (76×26mm). Plaques of SRBC were counted by a colony counter (Sekisui Chemical Corp., Osaka, Japan).

### 4. Expression of IL-1β and IL-6 mRNA

### 4.1. Extraction of RNA

### a) Spleen cells

Similarly to the method for PFC described above, the ABM extract (25 mg/kg) or PBS was intra-abdominally injected into mice. Four days later, spleens were excised, cut on steel mesh, and passed through a layer of gauze to obtain a suspension of spleen cells. The total cell RNA_was extracted from the spleen cell suspension by using an acid guanidium thiocyanate-phenol-chloroform (AGPC) method.

### b) Peritoneal cells

Macrophage culture was established from a leach liquor collected by washing using 10 mL of cold PBS. The leach liquor was pooled in a plastic tube and centrifuged at 250xg for 10 minutes. The cells in the leach liquor became pellets. The pellets were again suspended in RPMI (Nikken Bio-medical laboratory Inc., Kyoto, Japan) compensated with bovine fetal serum (FCS)(JRH Biosciences Inc., Lenexa, KS). The aliquot of abdominal cells (5×10⁵ cells/0.1 ml/well) was cultured in a flat-bottomed 96 wells culture plate (Becton Dickinson Co. Ltd, MA, US), with and without 0.1 or 1 mg/mL ABM extract. The culturing was performed at 37°C in a humidified atmosphere including 5% CO₂. After 24 and 48 hours, the total RNA was isolated by using the AGPC method.

### 4. 2. RT-PCR

The total RNA was transcribed to cDNA with an MLV reverse transcriptase (Life Technologies Ltd., MD, US). Oligonuleotide primer was constructed from housekeeping genes from cDNA sequences of IL-1β (250 bp), IL-6 (290 bp) and β-actin (268bp), which were published. Then, PCR was performed by, 30 cycles for IL-1β, and 32 cycles for I1-6, using the following primer pairs: β-actin sense (5'-GCATTGTTACCAACTGGGAC-3') and β-actin antisense (5'-TCTCCGGAGTCCATCACAAT-3'); IL-1β sense (5'-AGCTACCTGTGTCTTTCCCG-3') and IL-1β antisense (5'-GTCGTTGCTTGGTTCTCCTT-3'); IL-6 sense (5'-GATGCTACCAAACTGGAGATAATC-3') and IL-6 antisense (5'-GGTCCTTAGCCACTGCTTCTGTG-3'). An amplification profile was constructed from denaturing at 94°C for one minute, primer annealing at 56°C for one minute, and extension at 72°C for one minute. After the last cycle, extension at 72°C was further performed for 10 minutes. Then the reaction was cooled to 4°C to finish. The PCR product was subjected to 30% polyacrylamide gel electrophoresis, and then visualized using ethidium bromide.

### 5. Analysis on surface antigens

Expressionof the surface antigens on the spleen cells was measured by a direct immunofluorescent method. The spleen cells (5x10⁵ cells/100µL) obtained with or without the ABM extract were stained at 4°C for 45 minutes with 0.5 µg as a final product of fluoresceine-isothiocyanate (FITC)-conjugated or phycoerythrin (PE)-conjugated mAb. FITC-conjugated anti-CD3, anti-CD4, anti-CD8, anti-CD25, anti-CDllb (Mac-1) mAb and PE-conjugated anti-CD19 mAb were obtained from PharMingen Inc.(San Diego, CA). FITC-conjugated class II and anti-B7-1 mAb were obtained from Caltag Inc. (San Francisco, CA). These cells were subjected to incubation, and then, were centrifuged twice at 490xg for 5 minutes using 1 mL of PBS. Thereafter, the resultant pellet was again suspended in 0.5 ml of PBS including 100 µg/mL of CaCl₂/MgCl₂, 0.01% sodium azide and 1% FCS. Next, the resultant solution was analyzed using an FAC Scan flow cytometer (Becton Dickenson Co., MA, US).

### 6. Statistical analysis

All values are represented based on average ± S.D. The comparison between the ABM treated group and the control group was performed by Student's t-test. P values smaller than 0.05 are defined as being significant.

### II. Results

### 1. Influence of ABM on antibody production

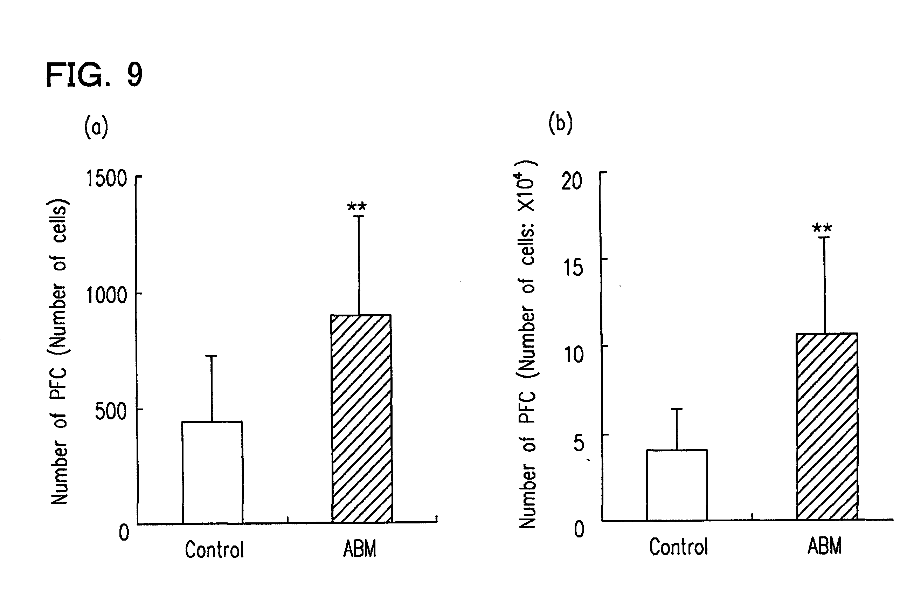

The influences of ABM on the number of PFCs per 10⁶ spleen cells and per spleen were examined. As shown in Table 1, there is no significant difference in the number of spleen cells between mice of the ABM-treated group (1.11 ± 0.29 × 10⁸ cells/spleen) and the control (0.98 ± 0.20 × 10⁸ cells/spleen). The number of PFCs per 10⁶ spleen cells was 425 ± 283 cells for the control and 897 ±440 cells for the mice of ABM treated group (Figure **9(a)**, Table 1). The number of PFCs per spleen was 3.8 ± 2.20 × 10⁴ cells for the mice of the ABM treated group and 10.5 ± 5.63 × 10⁴ cells for the control group (Figure **9(b)**, Table 1). As can be seen from these results, the ABM extract caused a significant increase in the number of PFCs per 10⁶ spleen cells and per spleen (p<0.01).

(The rest of the page is intentionally left blank.)

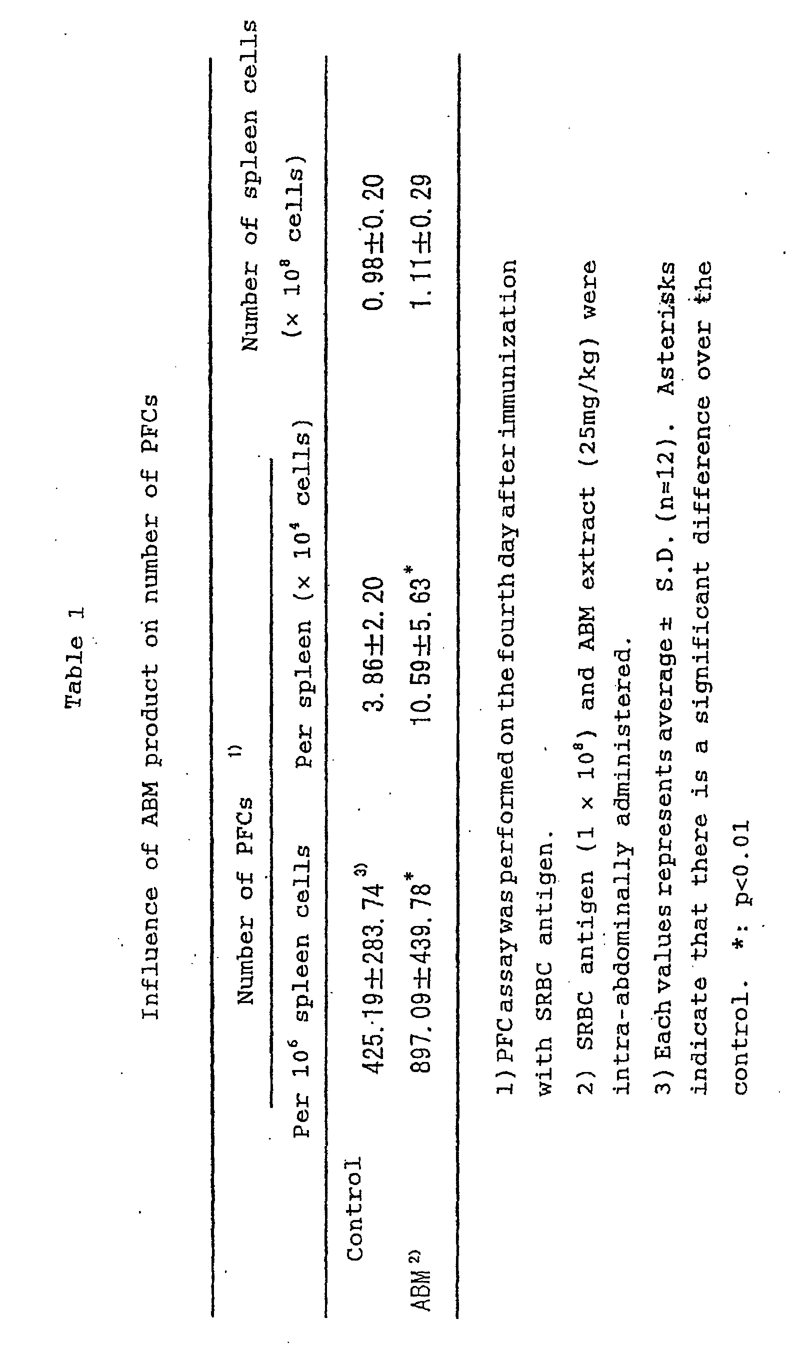

### 2. Influence of ABM extract on expression of IL-1β and IL-6 mRNA in spleen cells or abdominal macrophages

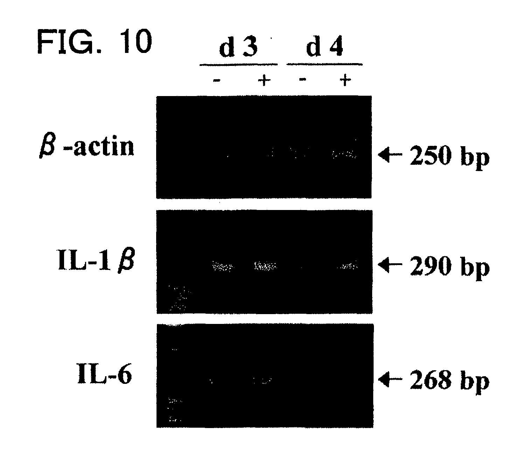

In order to examine how the ABM extract increased the PFCs, mRNA expression of IL-1β and IL-6 were examined. IL-1β and IL-6 are known as cytokines which differentiate B cells into antibody producing cells. Expression of IL-1β and IL-6 mRNA in the spleen was enhanced in the ABM extract treated group on the third day and the fourth day (Figure **10**). Especially, expression of IL-1β mRNA was enhanced most in the mice of the ABM treated group on the fourth day. In order to examine whether the ABM extract activated macrophages or not, induction of IL-1β and IL-6 mRNA expression was examined. As a result, it was found that the ABM extract strongly enhanced expression of IL-1β and IL-6 in a dose-dependent manner during the culturing after 24 hours (Figure **11**).

### 3. Influence of ABM extract on surface antigen of spleen cells

The percentages of Mac-1, B7-1, or CD25 antigen positive cells in mice of the ABM treated group were respectively 25.4 ± 1.4, 42.0 ± 2.0, or 26.9 ± 2.5. In the mice of the control group, they were respectively 17.0 ± 3.6, 42.0 ± 2.9, or 13.1 ± 2.2. There was a significant increase in the mice of the ABM treated group compared to the mice of the control group ( respectively, p<0.01, p<0.01, and p<0.001). However, no significant difference was recognized in percentages of CD3, CD4, CD8, CD19, or class II antigen positive cells in the two groups (Table 2).

(The rest of the page is intentionally left blank.)

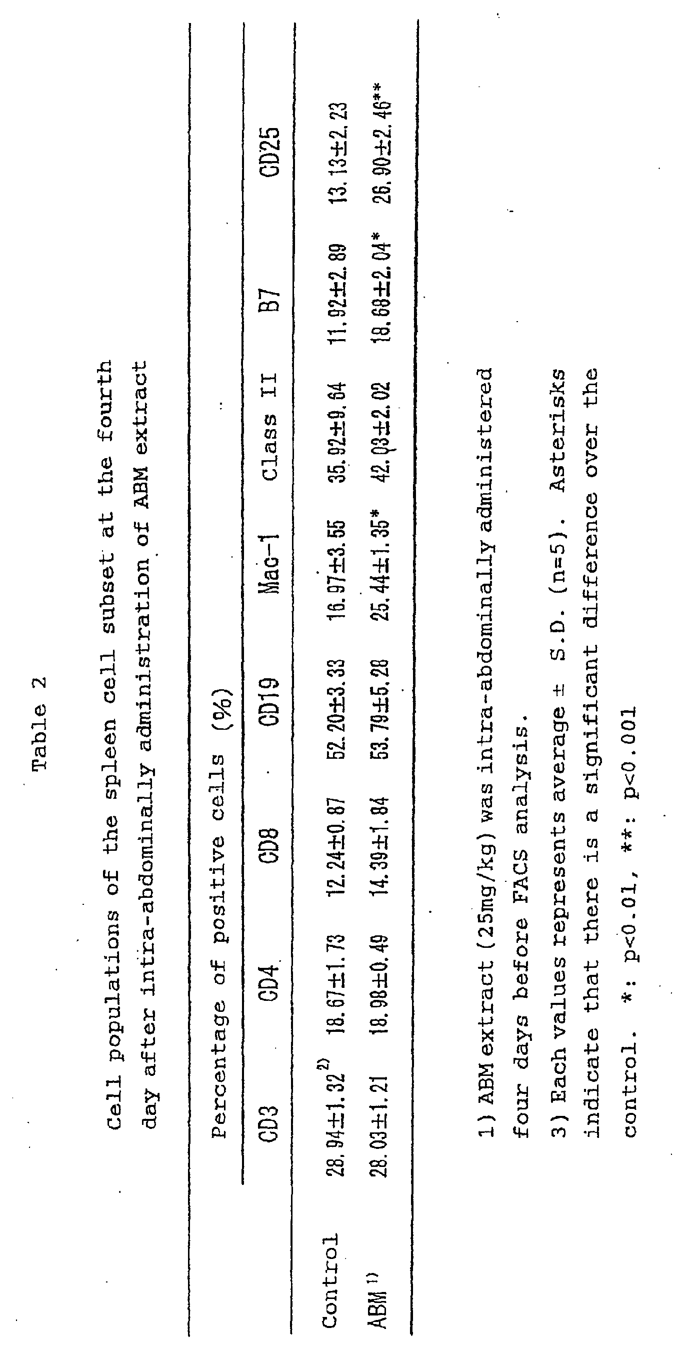

### 4. Discussion

It is widely recognized that ABM has antitumor activity (Fujimiya Y et al., ibid; Ebina T et al, ibid; and Mizuno M et al., ibid). There has been no report on the influence of ABM on antibody production. The present invention has shown that the ABM extract may enhance responsivity of the immune system. PFC assay was used for confirming effects of the ABM extract on antibody production. PFC assay is a typical method for examining humoral immune response (Cunningham AJ et al., ibid).

The number of PFCs in a spleen against SRBC antigen in the mice of the ABM treated group was increased by about three times that in the mice of the control group. However, there is no difference in the number of a population of B cells and spleen cells. In the spleens of the mice on the fourth day after immunization, the percentage of Mac-1 or CD25 antigen positive cells are significantly increased in the mice of ABM treated group compared to the mice in the control group. However, there was no significant difference in the population of CD 19 antigen positive cells between the two groups. Mac-1 antigen is present on differentiated monocytes/macrophages, granulocytes, and activated T lymphocytes. CD25 antigen is present on cell surface membranes of activated or differentiated T/B lymphocytes and differentiated monocytes/macrophages. CD19 antigen is present on cell surface membranes of non-activated B lymphocytes. Accordingly, these results suggest that the ABM extract mainly stimulates macrophages and T cell, and cause B cells to be differentiated without an increase in the number of B cells. Mizuno et al. (ibid) show that percentages of Thy-1, 2, CD4 and CD8 positive cell groups significantly increases in mice orally given a fraction from ABM which is soluble in hot water compared to control. However, the present inventors could not find a significant difference in the percentages of CD4 and CD8 positive cells. These mismatches may due to differences in the methods for extracting the ABM extracts, the administration routes, and concentrations.

Differentiation of B cells requires synergic effects of various cytokines secreted from macrophages or T cells (Mitsuzumi H et al., Jpn.J.Pharmacol.78(2):169-79). Antibody production against SRBC antigen has been reported to be decreased in Il-1^{-/-} mice because IL-1 activates T cell priming for antibody production (Nakae S et al. (2001), J.Immunol.167(1), 90-7). It is shown that GM-CSF has an influence on a mature functional APC and also results in enhanced response of an immune system in vitro by enhancing secretion of IL-1 and expression of Ia antigen (Morrissey PJ et al. (1987), J.Immunol.15;139(4):1113-1119). It is shown that IL-6 accelerates terminal differentiation of B cells into antibody secreting cells, and the main producers are monocytes and T cells (Morgan EL et al. (1990), J.Immunol.1;144(7):2499-505). The present application has shown that the ABM extract enhances expression of IL-6 and IL-1β mRNA from abdominal macrophages. Further, the same results are shown in expression of IL-1β and IL-6 mRNA of the spleen cells from the mice of the ABM treated group. These results have shown that enhancement of the antibody production by the ABM extract is induced by production of IL-1β and IL-6 from activating T cells and macrophages, and B cells are differentiated. As described above, the hot water extract from ABM is demonstrated to be an important substance for enhancement of the immune system and the prevention of various diseases.

### 5. Conclusion

The present inventors examined an immune enhancing activity of a hot water extract from dried agaricus (ABM). An influence of ABM on antibody production was examined by a plaque formation cell (PFC) method with respect to a sheep red blood cells (SRBC) antigen. The ABM extract significantly increased the number of PFCs in a spleen with an abdominal administration dosage of 25 mg/kg compared to control. A surface antigen on these spleen cells was analyzed usingflow cytometry. In comparison with control mice, Mac-1 or CD 25 positive cell populations increased, but there is no difference in CD19 positive cells. Expression of I1-6 and IL-1β mRNA was analyzed by RT-PCR. The expression was enhanced by the ABM extract in both abdominal macrophages and spleen cells. These results suggest that ABM extract is an effective stimulating factor for T cell and macrophage to release IL-1β and IL-6 and enhances production of antibody against the SRBC antigen.

### INDUSTRIAL APPLICABILITY

According to the present invention, by performing extraction of agaricus with hot water, a composition for preventing damage of a living body which is caused by an external factor and health foods including the composition are provided. The agaricus hot water extract includes an effective stimulating factor for T cells and macrophages to release IL-1β and IL-6. Thus, a composition or health food which enhances a capability of producing antibodies against an exogenous antigen in a living body is provided.

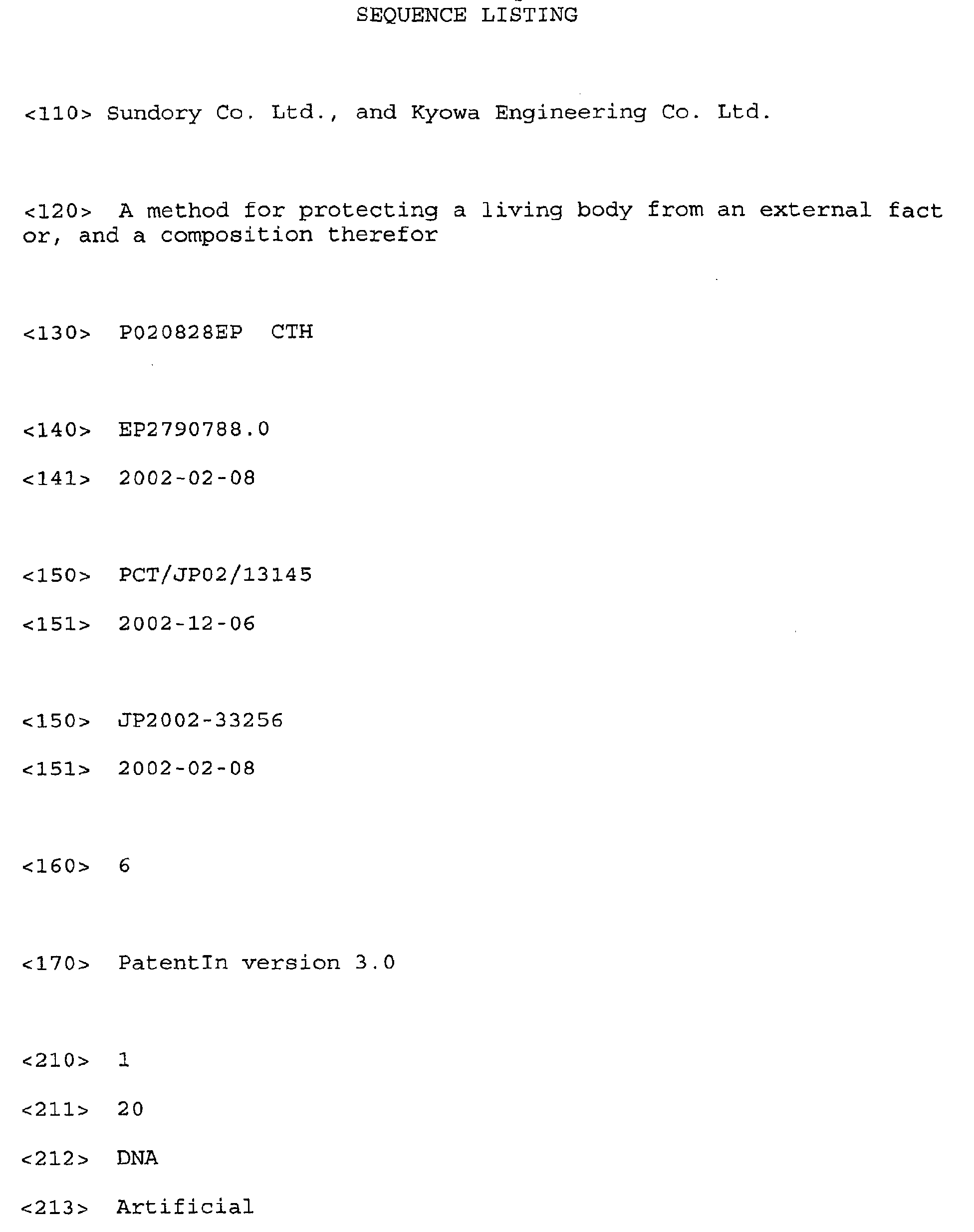

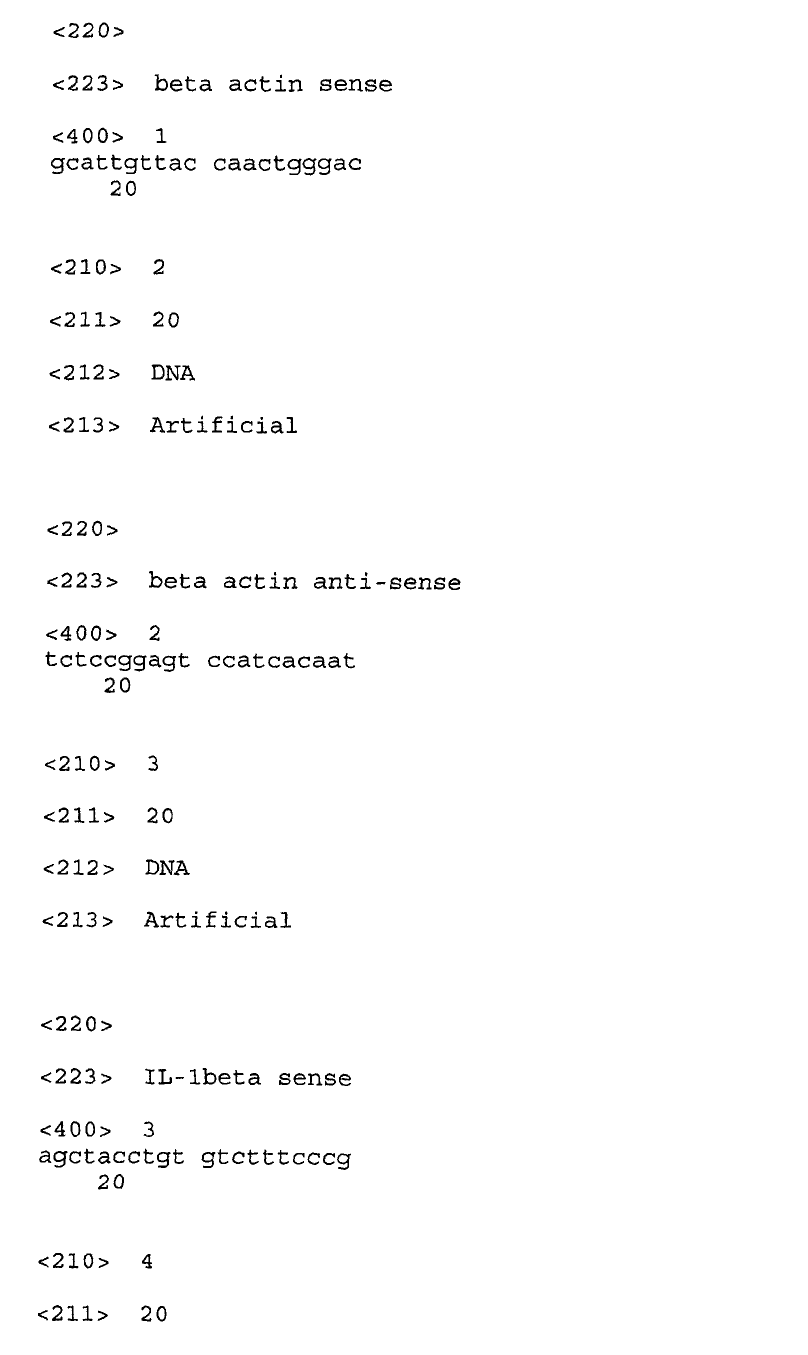

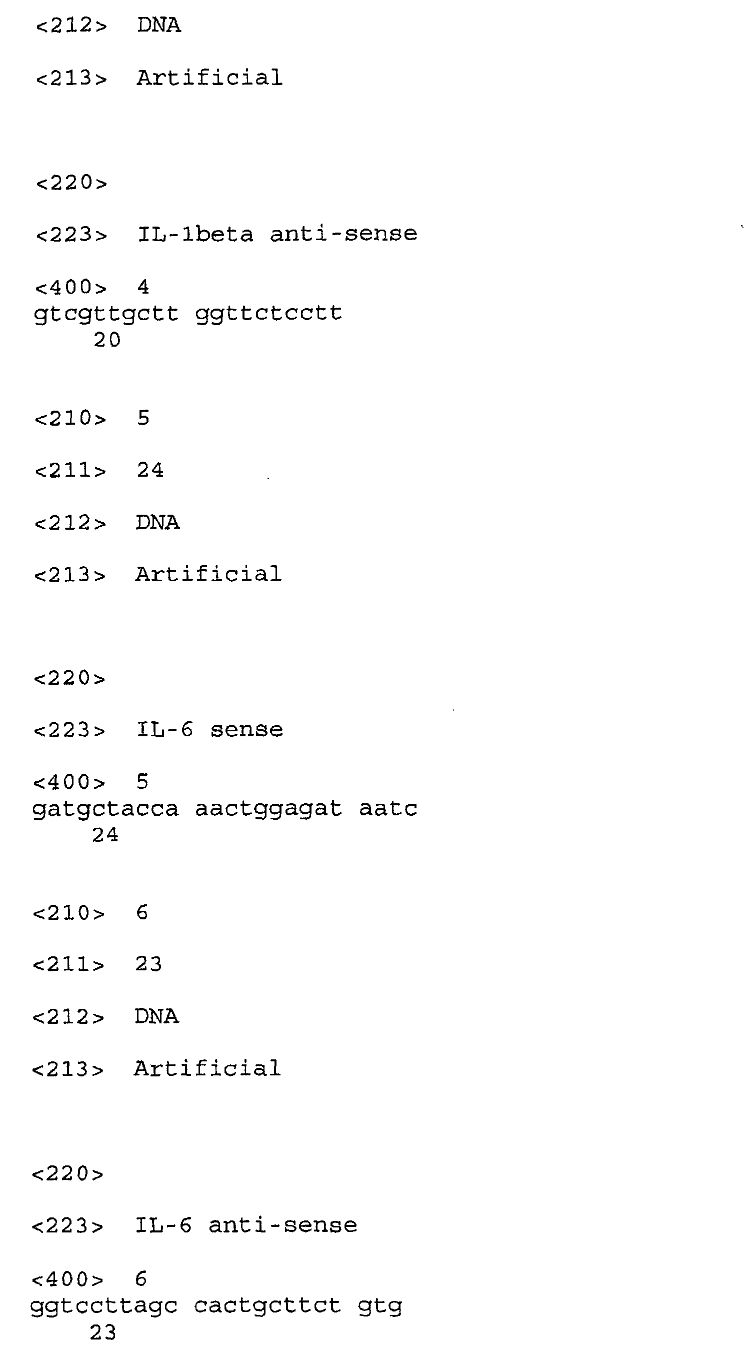

## Claims

1. A composition for protecting a living body from an external factor, which includes an extract from agaricus.

2. A composition according to claim 1, which protects a living body through immunocompetent cells.

3. A composition according to claim 2, wherein the immunocompetent cells are macrophages.

4. A composition according to claim 2, wherein the immunocompetent cells are macrophages or T cells.

5. A composition according to claim 2, wherein the immunocompetent cells are antibody producing cells.

6. A composition according to claim 2, which protects a living body through an enhancement of humoral immunity.

7. A composition according to claim 1, wherein the external factor is taken by smoking.

8. A composition according to claim 7, which protects a living body by decreasing genetic damage.

9. A composition according to claim 8, wherein the genetic damage is oxidative DNA damage of a pulmonary tissue.

10. A composition according to claim 1, wherein the external factor is an antigen substance.

11. A composition according to claim 6, wherein the enhancement of the humoral immunity is caused by activating lymphocytes selected from the group consisting of activating T cells and macrophages.

12. A composition according to claim 11, wherein the enhancement of the humoral immunity is caused by enhancing expression of interleukins selected from the group consisting of interleukin-1β and interleukin 6.

13. A composition according to claim 1, further comprising a pharmaceutically acceptable carrier.

14. A composition according to claim 1, which is in a form selected from the group consisting of powder, liquid, tablet, capsule, and pellet.

15. A method for protecting a living body from an external factor, which comprises a step of administering a composition including an extract from agaricus to a subject.

16. A method according to claim 15, which protects a living body through immunocompetent cells.

17. A method according to claim 16, wherein the immunocompetent cells are macrophages.

18. A method according to claim 16, wherein the immunocompetent cells are macrophages or T cells.

19. A method according to claim 16, wherein the immunocompetent cells are antibody producing cells.

20. A method according to claim 16, which protects a living body through an enhancement of humoral immunity.

21. A method according to claim 15, wherein the external factor is taken by smoking.

22. A method according to claim 21, which protects a living body by decreasing genetic damage.

23. A method according to claim 22, wherein the genetic damage is oxidative DNA damage of a pulmonary tissue.

24. A method according to claim 15, wherein the external factor is an antigen substance.

25. A method according to claim 20, wherein the enhancement of the humoral immunity is caused by activating lymphocytes selected from the group consisting of activating T cells and macrophages.

26. A method according to claim 25, wherein the enhancement of the humoral immunity is caused by enhancing expression of interleukins selected from the group consisting interleukin-1β and interleukin 6.

27. Use of an extract from agaricus for preparation of a composition for protecting a living body from an external factor.
